Europäisches Patentamt

European Patent Office

Office européen des brevets

(11) Publication number: **0 273 871 B1**

# EUROPEAN PATENT SPECIFICATION

(45) Date of publication of patent specification: **18.12.91**  (51) Int. Cl.⁵: **A61F 2/36, A61B 17/58**

(21) Application number: **87830131.6**

(22) Date of filing: **10.04.87**

(54) A femoral neck hip prosthesis.

(30) Priority: **02.12.86 IT 2254286**

(43) Date of publication of application:
**06.07.88 Bulletin 88/27**

(45) Publication of the grant of the patent:
**18.12.91 Bulletin 91/51**

(84) Designated Contracting States:
**BE CH DE ES FR GB LI NL SE**

(56) References cited:
EP-A- 0 149 527      EP-A- 0 196 258
EP-A- 0 217 034      EP-A- 0 238 438
WO-A-85/03426        GB-A- 2 063 676

(73) Proprietor: **G. CREMASCOLI S.p.A.**
**Via Clemente Prudenzio, 14/16**
**I-20138 Milano(IT)**

(72) Inventor: **Cremascoli, Patrizio**
**Via Clemente Prudenzio, 14/16**
**I-20138 Milano(IT)**

(74) Representative: **Cicogna, Franco**
**Ufficio Internazionale Brevetti Dott.Prof.**
**Franco Cicogna Via Visconti di Modrone,**
**14/A**
**I-20122 Milano(IT)**

**Description**

The present invention relates to a femoral neck hip prosthesis.

As is known, in the production of hip prostheses it is currently necessary to make differently shaped left and right femoral pins to obtain the desired anatomical shape for the left and right hips respectively. Moreover, because there is a range of different hip sizes and shapes to be accommodated the known types of pins, which are integrally formed, must be available in a large number of sizes and shapes, depending both on whether they are to be used in a right or left femur, and on the different anatomical conformations they are to assume.

This fact means that hip prostheses must in practice be made to measure, with a considerable expenditure of time and with the necessity of having available a very large reserve store in such a way as to be able to satisfy readily at least some of the requirements.

The EP-A-0 196 258 reference discloses a femoral neck hip prosthesis having substantially the features of the preamble of the main claim.

The object of the invention is to overcome the above-mentioned disadvantages by providing a hip prosthesis which is constituted by a simple base element which can be adapted to assume the desired anatomical conformation immediately prior to its use.

According to the present invention, therefore, there is provided a femoral neck hip prosthesis for a human hip, having the features of the characterizing part of the main claim.

The advantage of the present invention is that it provides a femoral neck hip prosthesis which, as well as improving the supply situation, allowing the whole of the wide range of required shapes and sizes to be immediately available, also offers the widest guarantees of reliability and safety in use.

Another advantage of the present invention is that of providing a hip prosthesis formed in such a way as to greatly facilitate the reparatory osteogenesis together with a greater possibility of a satisfactory outcome of the operation.

A major advantage of the present invention is that it makes it possible to assemble a hip prosthesis from a number of separate standard elements, which can be easily and rapidly assembled together at the moment of use without creating particular practical problems.

Two embodiments of the invention will now be more particularly described, by way of example, with reference to the accompanying drawings, in which:

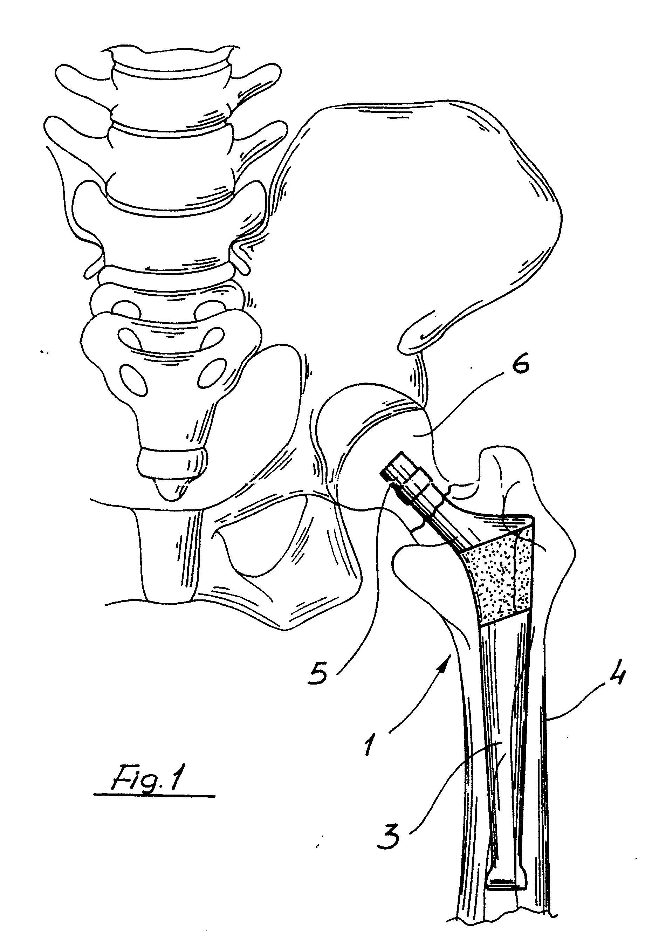

Figure 1 is a schematic view showing the hip prosthesis of the invention fitted in the bone;

Figure 2 is a perspective view showing the prosthesis of the invention with two different modular inserts usable therewith;

Figure 3 is a front view of the prosthesis showing a modular insert fitted to the central stem of the prosthesis;

Figure 4 is a side view of the prosthesis of the invention;

Figure 5 is a side view of the prosthesis from the same direction as Figure 4, with a modular adaptor for use as a left hip fitted thereto;

Figure 6 is a side view of the prosthesis from the same direction as Figure 4, with a modular insert for use as a right hip fitted thereto;

Referring now to the drawings, the embodiment of Figures from 1 to 6 comprises a hip prosthesis which is generally indicated with the reference numeral 1, constituted by a basic central stem 2 having an elongate, tapering shaped body 3 which can be inserted into the femoral bone 4. At an upper end of the body 3 is a shaped head 5 which projects upwardly and at an angle to be inserted, in a known way, into the head 6 of the femoral bone.

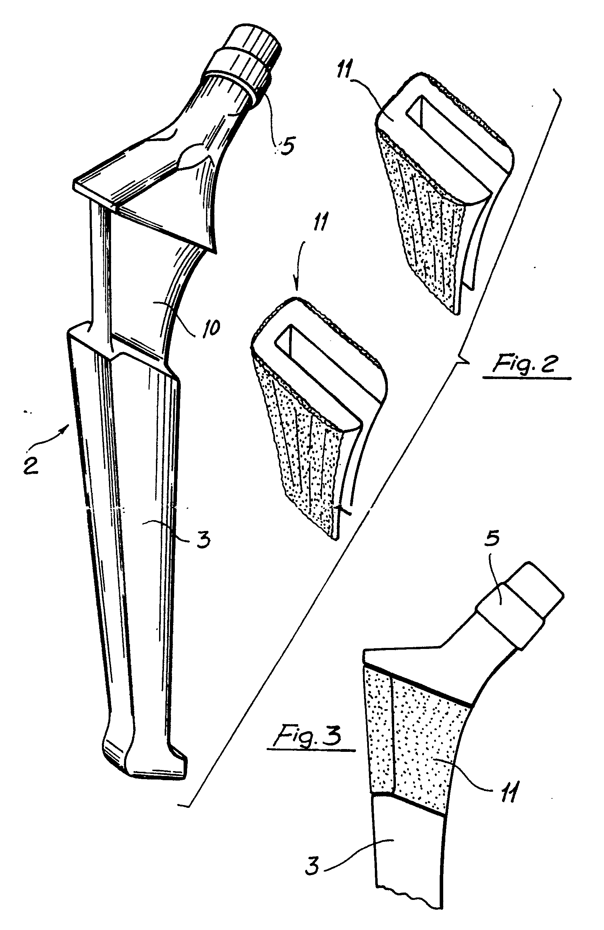

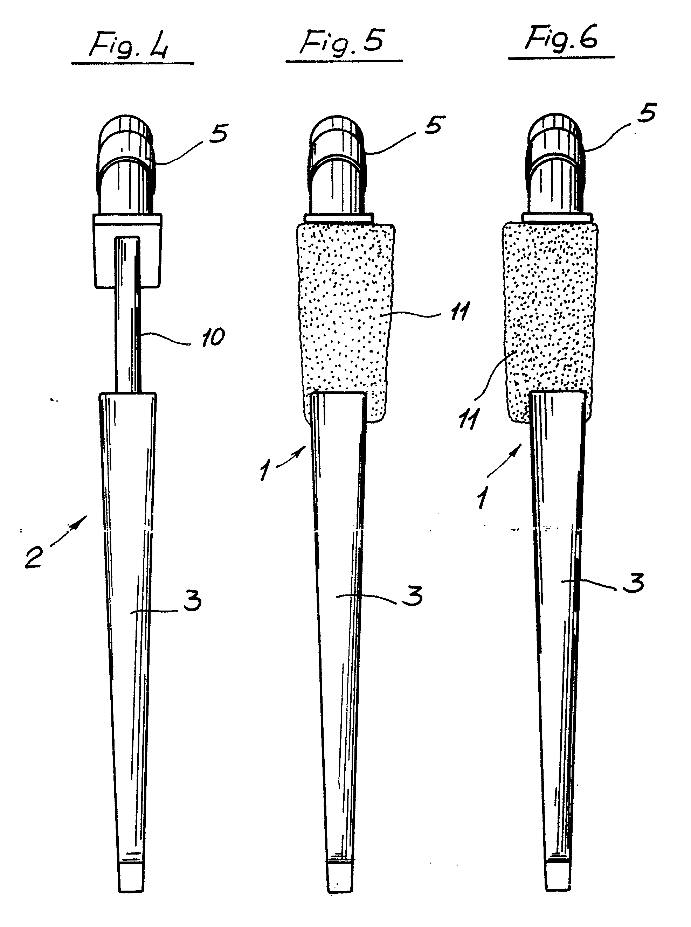

The body 3, close to the upper or head end, has a rebated section forming a seat 10 into which can be fitted modular inserts 11 of different sizes and shapes which, in transverse section, have a substantially U-shape as can be seen in Figure 2. Different inserts have limbs of different thickness, as can be seen in Figure 2 and in Figures 5 and 6 so as to form a right or left femoral pin when fitted to the basic central stem.

The said modular inserts 11 can have any shape so as to allow any desired anatomical form of the prostheses to be achieved, whilst starting from a single basic component, that is the basic central stem 2, which is not modified but merely carries different inserts to convert the prosthesis assembly thus formed into a form suitable for a left hip or a right hip.

The modular inserts 11 are preferably made of ceramic material and the mechanical coupling by which they are fitted into the seat 10 can be of any type in dependence on the various requirements of the individual case. From the practical point of view the important feature is that the modular inserts are firmly fixed in the seat 10 and secured to the basic central stem 2 forming a solid immovable composite body which offers the necessary guarantees of security.

With the above-described arrangement, that is to say utilising a hip prosthesis formed by means of two or more modular component s which can be assembled together for use, there is the possibility of standardising the production by basing it on a common central stem to which it is possible to fit different modular inserts so as to allow the desired anatomical configuration to be obtained without having to have available a wide range of different prostheses.

## Claims

1. A femoral neck hip prosthesis for a human hip, comprising a basic central stem (2, 2') having a body (3) adapted to be inserted into the femoral bone, and a head (5) adapted to be inserted into the head of the femur, the body (3) having at least one recess (10) for receiving a modular insert (11,), selected from one of a plurality thereof of different shapes and sizes, whereby to form a composite prosthesis in which the basic central stem is adapted for use as a right or a left femoral pin, characterized in that the modular inserts (11,) are substantially U-shape in cross section, and that said recess (10) has a central limb shaped to receive such an insert (11,).

2. A femoral neck hip prosthesis according to Claim 1, characterized in that said U-shaped insert comprises two limbs of different thickness.

3. A femoral neck hip prosthesis for a human hip according to Claim 1, characterized in that the said central stem (2, 2') is made of metallic material and the said modular inserts (11,) are made of ceramic material.

## Revendications

1. Prothèse du col du fémur pour fémur humaine, comprenant un fuseau médian de base (2, 2') présentant un corps (3) apte à être inséré dans l'os fémoral, et une tête (5) apte à être insérée dans la tête du fémur, le corps (3) présentant au moins un évidement (10) pour accueillir une insertion modulaire (11) choisie parmi plusieurs · formes et mesures différentes, de façon de former une prothèse composite où le fuseau central est apte à être employé comme tourillon fémoral droit et gauche, carachérisée en ce que les insertions modulaires (11) sont essentiellement en forme d'U dans leur section transversale, et en ce que ledit évidement (10) présente une saillie médiane formée de façon d'accueillir ladite insertion (11).

2. Prothèse du col du fémur selon la revendication 1, caractérisée en ce que ladite insertion en forme d'U comprend deux saillies ayan une épaisseur différente.

3. Prothèse du col du fémur pour fémur humaine, selon la revendication 1, caractérisée en ce que ledit fuseau central (2, 2') est fait d'un matériel métallique et lesdites insertions modulaires (11) sont faites d'un matériel céramique.

## Patentansprüche

1. Schenkelhalsprothese für menschliche Oberschenkel, mit einem inneren Grundstengel (2, 2'), der einen Körper (3) aufweist, der zur Einfügung in dem Schenkelbein geeignet ist, und einer Kopfteile, (5), die zur Einfügung in der Schenkelkopf geeignet ist, wobei der Körper (3) wesentlich eine Aufnahme (10) zum Aufnehmen eines modularischen Einführungsstück (11) verseht, das aus mehreren Formen und Grösse gewählt ist, um so eine zusammengesetzte Prothese zu bilden, wo der innere Stengel zur Verwendung als rechte oder linke Schenkelstift geeignet ist, dadurch gekennzeichnet, dass die Einführungsstücken (11) wesentlich einen U-förmige Querschnitt aufweisen und dass die obengenannten Aufnahme (10) mit einer mittleren Vorsprung vorgesehen ist, die zum Aufnehmen des obengenannten Einführungsstück (11) geeignet ist.

2. Schenkelhalsprothese nach Anspruch 1, dadurch gekennzeichnet, dass das obengenannte U-förmige Einführungsstück mit zwei Vorprünge verschiedener Gewicht vorgesehen ist.

3. Schenkelhalsprothese für menschliche Oberschenkel, nach Anspruch 1, dadurch gekennzeichnet, dass der obengenannte innere Stengel (2, 2') aus Metallstoff gemacht ist und die obengenannten modularischen Einführungsstücke (11) aus keramische Stoff gemacht ist.

_Fig.1_

Fig. 2

Fig. 3

Fig. 4   Fig. 5   Fig. 6